# EUROPEAN PATENT SPECIFICATION

(11) **EP 4 788 274 B1**
(45) Date of publication and mention of the grant of the patent: **09.12.2026**
(21) Application number: 25838831.3
(22) Date of filing: 19.12.2025
(51) Int. Cl.: A61F 5/44, A61F 5/445

(54) **OUTLET ELEMENT FOR AN OSTOMY APPLIANCE**
AUSLASSELEMENT FÜR EINE STOMAVORRICHTUNG
ÉLÉMENT DE SORTIE POUR APPAREIL D'OSTOMIE

(30) Priority: 20.12.2024 US 202463736769 P; 24.03.2025 GB 202504256
(43) Date of publication of application: 12.08.2026
(60) Divisional application: 26212762.4 / 4 846 987
(73) Proprietor: ConvaTec Limited, Deeside, Flintshire CH5 2NU (GB)
(72) Inventor: DAM-NIELSEN, Lena, Deeside, Flintshire CH5 2NU (GB); CURILA, Vladimir, Deeside, Flintshire CH5 2NU (GB); NIELSEN, Per, Deeside, Flintshire CH5 2NU (GB); HILL, Monica, Deeside, Flintshire CH5 2NU (GB)
(74) Representative: Wilson Gunn
(86) International application number: PCT/GB2025/060060
(87) International publication number: WO 2026/132831

(56) References cited:
- WO-A1-2018/098242
- WO-A1-2019/221830
- DE-A1- 102014 104 737
- US-A1- 2021 259 874

## Description

### Technical Field of the Invention

The present invention relates to an outlet element for ostomy appliances. In particular a closeable outlet element for draining an ostomy appliance. The invention also particularly relates to a closure element for the outlet element. Most particularly, the invention relates to outlet elements for ileostomy appliances and ileostomy appliances comprising an outlet element.

### Background to the Invention

An ostomy appliance may be provided with an outlet element for draining liquid and semi-liquid contents from teh bag of the ostomy appliance, either for direct disposal or via an additional appliance such as a drain bag which may be attached to the outlet element. To assist in draining, outlet elements are typically provided at the bottom of the bag such that gravity drives the contents of the bag towards and out of the outlet element when required.

This is particularly important for ileostomy appliances which are for connection to an ileostomy, which is a stoma formed from the small intestine. Due to the connection to the small intestine, stomal output is more frequent than a stoma formed from the large intestine (colostomy). In addition, the stomal output has a high liquid content and is generally a mixture of liquid and smaller solid chunks.

This necessitates careful design of the outlet element to ensure that it can be sealed securely to prevent unwanted leaks of liquid or odour from the bag but also be relatively easy to open and re-close again while being suitable for allowing solids to flow through the outlet with the liquid output. Some existing outlet elements use plugs which may be engaged with an outlet tube to stop fluid flow through the outlet element. These generally seal the tube via a push-fit or interference fit.

PCT/US2017/062964 discloses a drainable ostomy system including an ostomy pouch having an internal collection area and an outlet for draining contents from the collection area, and an interface assembly. The interface assembly includes an outlet body operably connected to the outlet and a cap configured for removable coupling with the outlet body.

While existing outlet elements can be effective, there remains a need to further optimise outlet elements in this field, particularly for users who have limited manual dexterity or other health complications that may make operation of the outlet element more difficult.

It is therefore an object of the present invention to at least partially address the above issues.

### Summary of the Invention

A first aspect of the present invention provides an outlet element for an ostomy appliance according to claim 1.

Advantageously, the opening is configured to allow the user to apply a force to the handling element with their fingertip to help remove the closure element from the outlet tube. This provides an additional feature that allows a user to open the outlet element in a more intuitive and easier way as their fingertip can engage the closure element and force then applied in a variety of ways. This allows the user to more easily open the outlet element, especially if they have limited dexterity as there is less reliance on being able to grip or pinch a part of the closure element to remove it.

Optional features of the outlet element of the first aspect are set out below. Any one or more of these features also apply to the outlet element described above in a broad sense. This includes any one or more of the optional features of this broadly described outlet element without requiring all features of the outlet element of the first aspect.

The opening may have a width/diameter of at least 12 mm. The width may be the maximum width measured between two points on opposite sides of the opening. This can help ensure the opening is big enough to receive enough of the user's fingertip to allow higher forces to be transferred from the fingertip to the closure element. The opening may have a width/diameter of at least 13 mm. This size is particularly preferred as it corresponds to the width of finger which 95% of female and male adult fingers exceed, as per Johnson et al, SJWEH Suppl 2007; (3); 26-32. The inventors have found that this size is thereby well suited to handling by the majority of users.

The opening may have a width/diameter of no more than 25 mm. This size helps ensure the handling element itself is not too bulky or does not add unnecessary weight to the ostomy appliance. It also allows most users to operate the handling element by wedging their finger into the opening either straight or at an angle without the need for tight gripping forces to be applied. The opening may have a width/diameter of no more than 20 mm. This size is preferred as it corresponds to the opening having a diameter that is no more than the width of 95% of female and male adult fingers, as per Johnson et al, SJWEH Suppl 2007; (3); 26-32. The inventors have found that the vast majority of users will be able to insert at least the tip of their finger into the opening with a relatively tight fit that helps them to apply force in an intuitive manner without gripping the handling element. The opening may have a width/diameter of no more than 15 mm. At this size, the majority of adult men and 50% of adult women have a finger width that is larger than this. Advantageously, the inventors have found this size facilitates the user in better operating the handling element as the can force just their fingertip into it and take advantage of the finger's natural elasticity, as well as elasticity of the opening if present, to grip the handling element.

The opening may have a width/diameter of 12 to 25 mm. The opening may have a width/diameter of 13 to 25 mm. The opening may have a width/diameter of 12 to 20 mm. The opening may have a width/diameter of 13 to 20 mm. The opening may have a width/diameter of 12 to 15 mm. The opening may have a width/diameter of 13 to 15 mm. These various combinations advantageously combine the benefits of the different ranges as mentioned above. The opening may have a width/diameter of 13 mm. This has been found to provide the optimum balance between being large enough to allow most people to insert part of their fingertip into the opening, while also small enough to ensure most people can generate a gripping force of the opening around their finger without needing to manually grip the handling element.

The sizes described above also have the advantage of being small enough such that a user can easily place their finger across or over the opening to push the closure element back into engagement with the outlet tube/end. The opening is sized so that this can be done easily without the fingertip entering the opening which may otherwise cause the closure element to become accidentally disengaged from the outlet tube when the user withdraws their finger.

The outlet element may have an axis extending therethrough. The axis may extend in a direction generally parallel to fluid flow through the outlet element. The axis may be provided centrally with respect to the tubular cross-section of the outlet element. The axis may be intended to be generally vertical in use. The outlet tube may comprise an axis. The handling element may comprise an axis. The axes of the outlet tube and outlet element may be parallel and preferably co-axial. The axes of the outlet tube and handling element when closing the outlet tube may be parallel and preferably co-axial.

The handling element may be resiliently deformable. The handling element may be formed of an elastically deformable material. The handling element may be configured to elastically deform upon insertion of a fingertip into the opening. For example, the opening may elastically deform from a generally circular shape to a generally ovular shape. The opening may be configured to elastically expand upon insertion of a fingertip into the opening. This provides the benefit of allowing the opening to dynamically change size to fit a variety of different fingertip sizes as well as actively grip the fingertip once it is received. These features are most beneficial when combine with the opening being no more than 25 mm, no more than 20 mm or no more than 15 mm in diameter. Additionally, as a user's fingertip cross-section is generally different for different users as well as an oval and not round, the opening with these features will be better able to conform to the shape of the fingertip.

The closure element may comprise a stop. The stop may be configured to abut the outlet end of the outlet tube. This helps control respective positioning of the closure element and outlet tube to ensure efficient closure and easy opening. The opening may be spaced apart from the stop. The opening and stop may therefore be spaced apart in a direction parallel to an axis of the outlet tube. The handling element may extend from the stop away from the outlet end, and preferably to the opening. The opening may be spaced apart from the stop by a distance of at least a third a diameter of the opening. This helps provide an opening spaced such that effective force can be transferred by the fingertip of a user to remove the closure element, for example a force away from the outlet tube end is required. The opening may be spaced apart from the stop by a distance of at least a half a diameter of the opening. This helps to facilitate better "gripping" of the user's finger due to deformation of the finger and/or opening. The opening may be spaced apart from the stop by a distance of at least a two-thirds a diameter of the opening. This can help ensure the opening is sufficiently spaced from the outlet tube to reduce the risk of leakage from the ostomy appliance contacting the user as the outlet element is opened using the handling element. The opening may be spaced apart from the stop by a distance of at least a diameter of the opening. These larger sizes provide further benefits of increased gripping but also easier locating of the handling element without looking at the outlet element.

The closure element comprises a plug configured to be received into the outlet tube. This provides a simple and robust way for the closure element to seal the end of the outlet tube by a push-fit or interference fit.

The plug may be tubular. The plug may comprise a capped end. This helps provide a simple and efficient design.

The plug may be configured to seal against an inside of the outlet tube. The plug may have a shape that matches an internal shape of the outlet tube. The plug may comprise an outer perimeter that is slightly larger than an inner perimeter of the outlet tube. Slight deformation of the plug and/or outlet tube may therefore provide the necessary forces to enable an interference fit when the outlet element is closed by the closure element. The outlet tube may be generally cylindrical at the outlet end. The plug may be generally cylindrical. This simple shape further provides advantages of rotational symmetry that makes the plug easier to fit into the outlet tube.

The plug and handling element are linearly arranged along the closure element. The plug and handling element are therefore one after the other along the closure element. A length of the closure element may be defined from a tip of the plug to the opening. The tip of the plug may be defined by the capped end of the plug. The length of the plug may be defined from the tip of the plug to a base of the plug. The handling element may be attached to the base of the plug. The handling element may be attached directly to the base of the plug. This provides a simple construction that enables intuitive use of the plug and handling element as the plug is essentially an extension of the handling element. The user can thereby close the outlet element by inserting the plug into the outlet tube.

The plug may converge towards the capped end. The plug may converge towards its tip. The plug may comprise a convergent shape. This can enable easier closing of the outlet element as the convergent shape may be used to guide the rest of the plug into the outlet tube.

The plug may comprise a conical end. The conical end may provide the convergent shape. The conical end may provide the capped end. This enables even easier closing as the conical shape helps guide the plug into the outlet element more efficiently and intuitively also provides an arrow shape to instruct the user on how to use the plug.

The plug, or conical end, may comprise a point at its tip. The plug may be blunted at its tip. The plug may be rounded at its stip. The convergent shape of the plug may be truncated. The conical end may be truncated. No more than 10%, no more than 5%, or no more than 3% of the area of the convergent shape, or conical end, may be truncated. At least 0.5%, or at least 1% of the area of the convergent shape, or conical end, may be truncated. The truncation may have a width of no more than 5 mm, or no more than 3 mm or no more than 2 mm. The truncation may have a width of at least 0.5 mm or at least 1 mm. The capped end may therefore not be pointed. This ensures there are no sharp points on the plug while maintaining ease of use due to the overall convergent shape.

The plug may comprise one or more sealing ribs. The one or more sealing ribs may be configured to seal against an inside of the outlet tube. The sealing ribs thereby provide additional sealing capabilities for the plug. The one or more sealing ribs may extend from an outside of the plug. The one or more sealing ribs may extend circumferentially around the plug, preferably all the way around. The sealing ribs may be sealing rings. This can help ensure they provide a fluid-tight seal between the plug and outlet tube.

The plug may comprise one or more sealing ribs provided along the length of the plug. The plug may comprise two or more sealing ribs, or three or more sealing ribs, or four or more sealing ribs. The plug may comprise five sealing ribs. This ensures a highly resilient and reliable seal. Where the plug comprises two or more sealing ribs, the sealing ribs may have different lengths measured out from the plug. The sealing ribs may be longer the closer they are to the handling element along the plug. The outlet tube may narrow in a direction away from the outlet end. The change in length of the sealing ribs may match the narrowing of the outlet tube. Thus, the sealing ribs and outlet tube are complimentarily shaped to ensure efficient sealing and improve ease of manufacture.

The sealing ribs may be equally spaced apart along the length of the plug. The sealing ribs may be provided along a region of the plug that has a constant outer perimeter. The sealing ribs may be spaced to cover approximately half the length of the plug. This can help spread the sealing force from adjacent ribs evenly to ensure better sealing.

The sealing ribs may be arranged generally towards a tip of the plug. The sealing ribs may be arranged generally away from the handling element. This helps ensure sealing is effected at a distance from the handling element to reduce the likelihood of leakage of stomal output onto the user.

The plug may comprise a proximal sealing rib. The proximal sealing rib may be provided towards the tip of the plug. The proximal sealing rib may be provided around, or adjacent to, a capped end of the plug. The proximal sealing rib may be provided around, or adjacent to, a base of the conical end of the plug. The proximal sealing rib can help to provide a first layer of sealing around the plug. The proximal sealing rib may be structurally supported by the capped end of the plug, or preferably by the conical end of the plug where present. This helps provide additional sealing capability and helps reduce passage of stomal output between the plug and outlet tube. The proximal sealing rib may be the shortest sealing rib where there are two or more sealing ribs.

The handling element may comprise a bore extending from the opening into the handling element. This provides additional depth to the opening to allow the fingertip to more effectively grip, and/or be gripped by, the opening. The bore may be cylindrical. The bore may extend from the opening through most of a length of the handling element. The bore may extend from the opening through the whole length of the handling element. The length of the handling element may be defined from the opening to the plug. This can help allow the sides of the handling element to flex and stretch around the opening to better accommodate the fingertip of a user. The bore may extend to the stop. The bore may extend through the entire handling element. This can help allow a user to insert their finger into the handling element and more securely operate the handling element without needing to grip it tightly. It also provides additional flexion when the handling element needs to stretch to accommodate the finger of a user.

The bore may extend into the plug. The bore may extend substantially the entire length of the closure element. The bore may extend from the opening to a capped end of the closure element. The bore may extend from the opening to a capped end of the plug. Of course, the bore would not extend through the whole length of the closure element, as otherwise it would provide a passage for stomal output through the closure element. This advantageously reduces the materials required for the closure element and provides a shape that is easily injection moulded without a propensity for defects.

The bore may comprise a step between the handling element and the plug. The step may correspond to the location of the stop. The plug may comprise a smaller inner perimeter (or diameter) than the handling element. This helps provide a simple and effective way of constructing the closure element with an integrated stop.

The closure element may comprise a wall. The wall may be tubular. The wall may define the shape of the handling element. The wall may define the shape of the plug. The wall may extend from the capped end to the opening. The sealing ribs may extend from the wall. The closure element may be hollow. The wall may have a substantially constant thickness over the plug. The wall may have a substantially constant thickness over the handling element. The wall may be thicker in the handling element than in the plug. This can ensure the closure element is easy to manufacture, and simultaneously provide the required mechanical properties and other features of the closure element.

The closure element may comprise a relatively thick wall region adjacent to the plug. The plug may have a wall thickness that is less than the relatively thick wall region, for example the plug may have a thickness of no more than 80%, no more than 70%, no more than 60%, no more than 50%, no more than 40%, or no more than 30% the thickness of the relatively thick wall region. The relatively thick wall region may be provided by the stop. The handling element may have a wall thickness greater than that of the plug. The handling element may have a wall thickness at least 25% greater than, at least 50% greater than, at least 75% greater than, or at least 100% greater than that of the plug. Thus, structural support is provided to the plug to help ensure it does not easily collapse during use and to ensure the sealing ribs operate effectively.

The handling element may comprise one or more flanges. The one or more flanges may be outwardly extending. As such, the one or more flanges may extend out from an outer surface of the handling element. The flanges may extend from the wall. The one or more flanges may extend out in a direction perpendicular to an axis of the handling element. Where the handling element has a tubular shape, it may define the axis of the handling element. The axis of the handling element may extend parallel to its length. The flanges advantageously provide additional features that can be easily gripped to allow the user to better manipulate the handling element or apply force to it in a more controlled manner.

The handling element may comprise a proximal flange. The proximal flange may be outwardly extending. The proximal flange may be provided at, or towards, an end of the handling element. The proximal flange may be provided around, or towards, the stop. The proximal flange may be adjacent to, or towards, the outlet tube when the outlet end is closed by the closure element. Advantageously, the proximal flange helps to prevent leakage of stomal output onto the user's hand/finger in use as it provides a lip that blocks leaks. It also physically helps separate the finger from the outlet tube which may contain stomal output on its outside surface.

The proximal flange may be positioned around the stop. This ensures that the proximal flange effectively demarcates where the handling element ends and where the outlet tube will reach along the closure element. This helps the user to ensure they do not touch the outlet tube accidentally.

The handling element may comprise a distal flange. The distal flange may be outwardly extending. The distal flange may be provided around, or towards, the opening. The distal flange advantageously provides a element the user can use to help apply force away from the outlet tube to remove the closure element from the outlet end. Additionally, the distal flange may provide a region around the opening that is less flexible compared to a mid-section of the handling element. This may be the case where the bore extends into the handling element. This means the user can insert their fingertip in past the opening, and the opening will then provide additional resistance to the user removing their fingertip. This allows better control and more force to be transferred to the handling element to remove the closure element from the outlet end.

The handling element may be cylindrical. The handling element may comprise a mid-section. The mid-section may comprise straight walls. The handling element may comprise a flange provided at either end of the mid-section, for example the proximal and distal flanges. This construction provides a simple and effective handling element, such as for the reasons mentioned above.

The handling element may curve outward to meet one or more of the flanges. Preferably, the handling element may curve outward to meet each flange. The handling element may curve outward to meet the proximal flange. The handling element may curve outward to meet the distal flange. The sides of the handling element may therefore be free of sharp corners. This provides a smoother outward appearance and feel to the handling element and can assist in helping it fit to a users finger when grasping the sides of the handling element. The handling element may curve outward to meet the proximal flange and the distal flange. This helps provide a "U" shape along the side of the handling element which more naturally receives the profile of a finger.

The outlet element may comprise a connector. The connector may connect the closure element to the outlet tube. The connector may be configured to prevent separation of the closure element and the outlet tube when the closure element is not closing the outlet end. This can help ensure the closure element does not get lost or become inaccessible to the user. It also helps in disposal or recycling as the parts of the outlet element are kept together.

The connector may comprise a lateral arm. The lateral arm may extend from the outlet tube at a connector location. The lateral arm may extend perpendicular to an axis of the outlet tube. The lateral arm may have a length of at least the distance between the connector location and the outlet end along the axis of the outlet tube. The lateral arm thereby facilitates movement of the closure element into a position where it can close the outlet end. Additionally, it helps separate the closure element from the outlet end when it is not closing the end to ensure the closure element does not block stomal output leaving the ostomy appliance or connection of the outlet end to other devices.

The distance from the connector location to the outlet end may be substantially the same as the length of the plug. This can help ensure the plug is more easily fitted into the outlet end by remaining aligned with the axial end during fitting.

The connector may comprise a curved section. The curved section may extend from the closure element. The curved section may connect to an end of the lateral arm distal from the outlet tube. The curved section may comprise an axial arm extending parallel to the axis of the outlet tube. The curved section may comprise a second lateral arm extending perpendicular to the axis of the outlet tube. The second lateral arm may be connected to the closure element. The axial arm may connect the lateral arm to the second lateral arm. The connector may be S-shaped. The axial arm may be shorter than the lateral arm. The second lateral arm may be shorter than the axial arm. As described above, the curved section may hold the closure element axially away from the outlet end. This helps ensure it does not come into contact with stomal output and is out of the way when not closing the outlet end. Additionally, the sizes of the arms are further controlled to provide these benefits.

The outlet tube may comprise one or more internal ridges. The outlet tube may comprise two internal ridges. The ridges may extend inwards from an inner surface of the outlet tube. The ridges may be configured to seal against the plug. The ridges may be configured to co-operate with the sealing ribs of the plug. The ridges may extend around the circumference of the outlet tube, preferably the entire circumference. This can help provide additional sealing action.

The outlet tube may be configured to connect to an external drainage device, for example an external tube. The outlet tube may be configured to receive an external drainage device inside the outlet tube. In such circumstances, the external drainage device may have a cross-sectional size/shape corresponding to that of the plug. The outlet element may be provided with an adaptor to be received into the outlet tube and provide an adaptor outlet that is smaller than the outlet tube. The adaptor may be generally annular in shape. This can help convenient attachment of smaller devices inside the outlet tube.

The outlet tube may be configured to receive an external drainage device around the outlet tube. The outlet end may be tapered. The outlet end may comprise a tapered section. This can help the outlet tube to be inserted into an external drainage device.

The outlet tube may provide a tortuous path for stomal output to exit between the outlet tube and a connected external drainage device. This ensures that liquid/stomal output is less likely to leak.

One or more features of the outlet element may be formed on the outlet tube by one or more strengthening ribs. These preferentially may be added to improve the structural strength of the outlet tube. The ribs may have a substantially constant thickness. The ribs may be formed onto the surface of the outlet tube. This can assist in ensuring the features are moulded with a lower risk of manufacturing defects. The one or more ribs may comprise circumferential ribs extending around the circumference of the outlet tube. Preferably the outlet element may comprise four circumferential ribs. Where there are two or more circumferential ribs, they may be equally spaced apart along the length of the outlet tube. The connector location may be provided into one of the circumferential ribs, preferably the circumferential rib furthest from the outlet end. The one or more ribs may comprise axial ribs extending along the axis of the outlet tube. The chamfered edge may be provided by one or more ribs, for example two or more spaced apart axial ribs, preferably four spaced apart axial ribs.

The one or more strengthening ribs may be spaced apart from the outlet end in a direction along an axis of the outlet tube. The one or more strengthening ribs may be spaced apart from the tapered section in a direction along an axis of the outlet tube. A recessed section may be provided between the tapered section and the one or more strengthening ribs. The recessed section may be substantially cylindrical. The recessed section may have a constant outer diameter. The recessed section may provide a region for receiving a connector of an external drainage device. The outlet end may be separated from the recessed section by a lip. The outlet end may widen towards the lip. The lip may comprise a step inwards in the outer profile of the outlet tube. The lip may constitute a reduction of at least 10% in the outer diameter of the outlet tube. The step may be configured to prevent a connector of an external drainage device from sliding off the outlet tube. The recessed section may have a length of about 7 mm. The lip may be provided between the tapered section and recessed section. Thus, the recessed section and lip help to ensure an external drainage device can efficiently attach to the outlet tube.

The outlet element may comprise a substantially constant internal perimeter or diameter along the outlet tube, for example from the milking region to the outlet end. The internal width or diameter of the outlet end may be at least 1 cm, or at least 1.2 cm or at least 1.3 cm. The internal width or diameter of the outlet end may be no more than 3 cm, or no more than 2 cm or no more than 1.5 cm. Preferably, the internal width or diameter of the outlet end may be 1.3 cm. This helps provide an outlet that is optimally sized for an ileostomy appliance and can handle the typical stomal output comprising both solid and liquid matter.

The outlet element may comprise an inlet end. The inlet end may be connected to an ostomy appliance, or configured to connect to the ostomy appliance. The inlet end may be configured to receive stomal output from a pouch of the ostomy appliance. The inlet end may be larger than the outlet end. The inlet end may comprise thicker walls than the outlet end. The inlet end may be ovular. The inlet end may have a lens shape. The major axis of the inlet end may be arranged parallel to the walls of the ostomy appliance, for example as described below. The inlet end may be configured to be provided between the two walls of the ostomy appliance. This can help ensure stomal output flows into the outlet element effectively and that the inlet end is robust and does not become easily detached from the ostomy appliance.

The connector may extend in a direction parallel to the major axis of the inlet end. The connector may be intended to extend parallel to the walls of the ostomy appliance. This can help provide a more compact shape to the ostomy appliance once the outlet element is attached to it.

The inlet end may comprise a tubular inlet. The tubular inlet may have a constant wall thickness. The tubular inlet may have a larger wall thickness than the milking region. This can help make the milking region more flexible than the tubular inlet. The milking region may have a wall thickness of no more than 95%, or no more than 90% the wall thickness of the tubular inlet. The milking region may have a wall thickness of at least 70%, or at least 80% the wall thickness of the tubular inlet. This helps ensure the milking region is more flexible while retaining sufficient strength not to break during use as well as sufficient similarity to the thickness of the tubular inlet to reduce the prevalence of artefacts during manufacture.

The tubular inlet has a length of at least 2 mm, or at least 4 mm, or at least 6 mm, or at least 10 mm. The tubular inlet has a length of no more than 20 mm, or no more than 15 mm, or no more than 10 mm. This helps provide a robust section for attachment to the ostomy appliance and minimises any potential damage to the seal with the ostomy appliance due to milking as mentioned below.

The inlet end may comprise two flaps. The flaps may extend outwardly from the inlet end. The flaps may extend parallel to the major axis of the inlet end. The flaps may be intended to extend between the walls of the ostomy appliance. The flaps may extend down the tubular inlet. The flaps may be tapered. The flaps thereby provide stronger sealing between the ostomy appliance and the outlet element.

The outlet element may comprise a milking region. The milking region may be provided between the inlet end and outlet tube. The milking region may be configured to allow a user to squeeze stomal output towards the outlet tube. This action is typically referred to in the art as "milking". The milking region may be tapered. The milking region may narrow in a direction towards the outlet tube. The milking region may be tubular. The milking region may comprise an oval cross section when viewed along the axis of the outlet element/outlet tube. The milking region may comprise a substantially constant minor axis. The milking region may comprise a narrowing major axis. This shape advantageously allows efficient squeezing or milking of the milking region along its minor axis to urge stomal output towards the outlet tube and out of the outlet element. Typically, such a milking region is only required in ileostomy appliances as urostomy appliances connected to the bladder only handle liquid and colostomy appliances do not produce output suitable for milking.

The milking region may comprise relatively flexible opposing walls. The walls may widen in a direction away from the outlet tube. The walls may be provided parallel to the major axis of the milking region. The walls may therefore be suitable for milking of stomal output towards the outlet tube. This helps the user to easily milk the outlet element, urging stomal output out of the tube, by squeezing the milking region.

The outlet element may be formed of one or more of a variety of materials, but preferably, it is formed from a plastics material. The outlet element may comprise a thermoplastic material. The thermoplastic material may have a melt flow index of 2.5 to 43 g/10min as measured according to standard ASTMD1238-10 at 190 degrees Centigrade and with a 2.16 kg mass applied. The outlet element may comprise a thermoplastic elastomer. Preferably, the outlet element is formed from the co-polymer ethylene vinyl-acetate (EVA), EVA being a co-polymer of ethylene and vinyl-acetate (VA). In one example, the EVA may comprise 18% by weight VA. This helps to provide an easily manufacturable outlet element that has the required structural properties. In other examples polyethylene may be used in the construction of the outlet element, for example low density polyethylene. Other suitable materials include thermoplastic polyurethane and Styrene-Ethylene-Butylene-Styrene plastics.

The outlet element may be formed of a material with a shore A hardness of at least 60, or at least 70, or at least 80. The shore A hardness may be measured according to the standard ASTM D2240-15 using a type A presser foot. The outlet element may be formed of a material with a Young's modulus of about 3 MPa to 4 MPa. This can help ensure the material is structurally strong and rigid enough to perform adequate sealing and connection to other external elements.

In another broad aspect of the present invention there is provided an ostomy appliance comprising an outlet element. The outlet element may be that described in broad terms above, and may have any of the features of an outlet element as described herein.

Accordingly, in a second aspect of the present invention there is provided an ostomy appliance comprising the outlet element of the first aspect above.

That is to say, according to the second aspect of the invention, there is provided an ostomy appliance comprising an outlet element according to claim 1.

The ostomy appliance of the second aspect may of course include any one or more of the optional features of the outlet element of the first aspect, or the features of an outlet element as described in broad terms herein.

The ostomy appliance may be an ileostomy appliance. That is to say, the ostomy appliance may include features rendering it particularly suitable for ileostomy. For example it may include a milking region as discussed above. A milking region is not necessary for urostomy pouches, which contain primarily/exclusively liquid output (urine), nor useful for colostomy pouches which contain primarily solid output which is not typically capable of being "milked" out of a relatively small diameter tube. As those skilled in the art will know, drainable colostomy bags normally have a drain which is formed integrally with the walls of the ostomy appliance, which can be pulled apart to produce a much larger outlet end, for example in the region of 7 cm in width which is much larger than the outlet ends used in ileostomy appliances which may be 1 to 2 cm in width.

The ostomy appliance may be a high-output ileostomy appliance. That is to say, the ostomy appliance may included features rendering it particularly suitable for high-output ileostomy. For example, it may have a capacity of at least 500 ml, and it may have a capacity of up to 1 L. Through use of the outlet element of the first aspect, the ileostomy appliance is easier and more hygienic to drain and additionally remains easy to connect to external drainage equipment like night bags which is important for ileostomy patients.

The ostomy appliance may comprise two walls. The two walls may be sealed around their periphery to form a pouch. The outlet element may be arranged to drain the contents of the pouch. The outlet element may be configured to receive stomal output from the pouch. The outlet element may be provided at or towards a bottom of the pouch. The outlet element may be provided between the two walls. The two walls may be sealed to the outside of the outlet element, preferably around the inlet end/tubular inlet. This helps provide efficient drainage of stomal output into and through the outlet element.

The two walls may be formed from a plastics material, such as a thermoplastic material, for example a thermoplastic elastomer. The two walls may be each formed of a plurality of layers of material. The two walls may be formed of polyvinylidene chloride (PVDC), EVA, VA or combinations thereof. Preferably, the walls and outlet element are formed from materials that may be welded together. For example, materials that may be heat welded together. This helps provide simple construction without unnecessary use of adhesives or other attachment devices.

The two walls may comprise a front wall and a rear wall. The front wall may be arranged to face away from a user in use. The rear wall may be arranged to face towards the user in use.

The rear wall may comprise a stomal inlet. The stomal inlet may be configured to receive the stoma of a user. The rear wall may comprise an adhesive wafer. The wafer may be configured to attach the ostomy appliance to the body of a user. The wafer may be provided around the stomal inlet. The wafer may therefore be annular. The wafer may comprise a hydrocolloid adhesive. The wafer may be attached directly to the rear wall. In other embodiments, a relatively rigid disc may be provided between the wafer and the rear wall. This can help provide a rigid object to assist in adhesion of the wafer to the body.

The rear wall may comprise a coupling element. The coupling element may be configured to attach to a corresponding coupling element provided on a separate base plate. The base plate may comprise the wafer. As such, the ostomy appliance may be a two-piece ostomy appliance.

The ostomy appliance may comprise a gas vent. The gas vent may be provided on the front wall. The gas vent may be configured to allow gasses to leave the ostomy appliance. The gas vent may comprise a deodorising filter, such as a carbon filter. This can help ensure the ostomy appliance does not inflate with gases.

### Detailed Description of the Invention

In order that the invention may be more clearly understood one or more embodiments thereof will now be described, by way of example only, with reference to the accompanying drawings, of which:
- Figure 1: is a front view of an outlet element for an ostomy appliance;
- Figure 2: is a front view of the plug of the outlet element of Figure 1 as indicated by detail A in Figure 1;
- Figure 3: is a top view of the outlet element of Figure 1;
- Figure 4: is a side view of the outlet element of Figure 1;
- Figure 5: is a cross-sectional view along line B-B of Figure 3 or C-C of Figure 4;
- Figure 6: is a rear view of an ostomy appliance including the outlet element of Figure 1 where the outlet element is open;
- Figure 7: is a cross-sectional view along line B-B of Figure 3 or C-C of Figure 4 where the outlet element is closed by the plug;
- Figure 8: is a front view of the outlet element of Figure 1 connected to an external drainage device;
- Figure 9A: is a schematic axial cross-section of a fingertip;
- Figure 9B: is a schematic side cross-section of the fingertip of Figure 9A;
- Figure 10A: is a schematic axial cross-section of the handling element of the outlet element of Figure 1;
- Figure 10B: is a schematic side cross-section of the handling element of Figure 10A;
- Figure 11A: is a schematic axial cross-section of the fingertip of the fingertip of Figure 9A inserted into the opening of the handling element of Figure 10A;
- Figure 11B: is a schematic side cross-section of the fingertip of the fingertip of Figure 9A inserted into the opening of the handling element of Figure 10A;
- Figure 12A: is a schematic axial cross-section of a large fingertip inserted into the opening of the handling element of Figure 10A;
- Figure 12B: is a schematic side cross-section of a large fingertip inserted into the opening of the handling element of Figure 10A;
- Figure 13A: is a schematic axial cross-section of a small fingertip inserted into the opening of the handling element of Figure 10A;
- Figure 13B: is a schematic side cross-section of a small fingertip inserted into the opening of the handling element of Figure 10A; and
- Figure 14: is an alternative cross-sectional view along line B-B of Figure 3 or C-C of Figure 4.

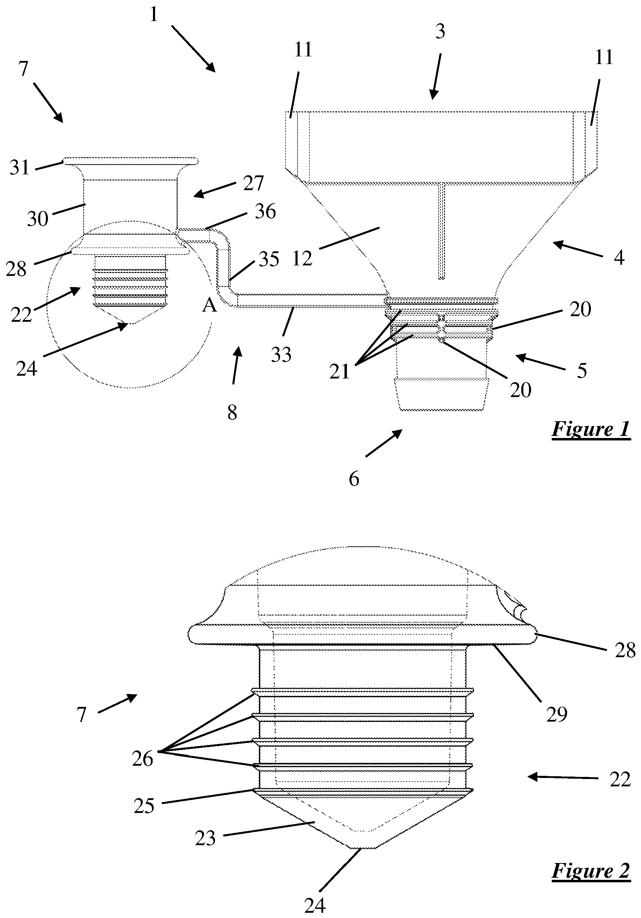

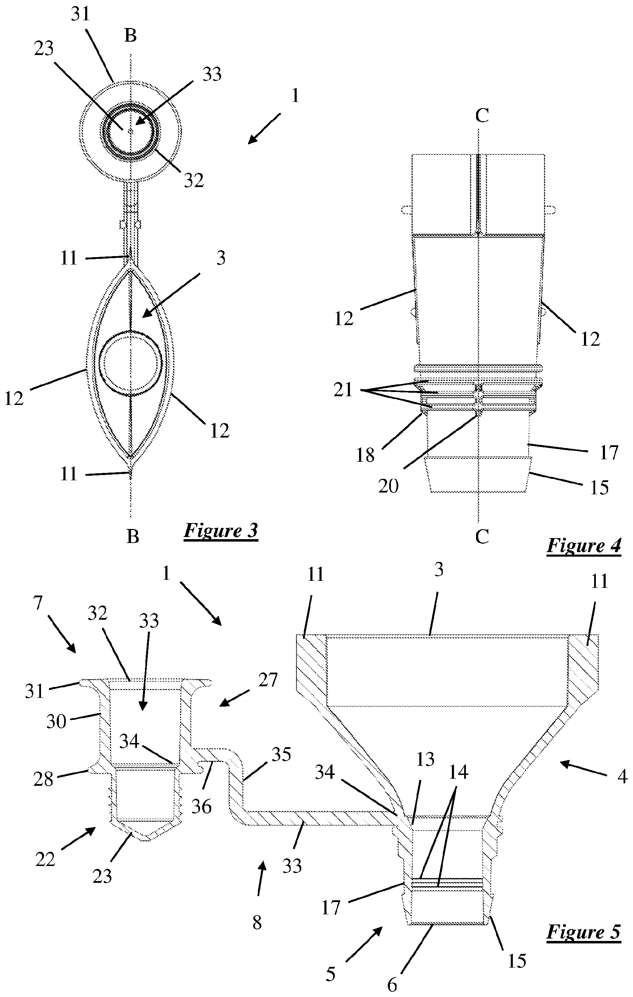

The invention relates to an outlet element 1 for an ostomy appliance 2. The ostomy appliance of this invention is an ileostomy appliance, in particular a "high-output" ileostomy appliance. Referring to Figures 1 to 5 and 14, the outlet element 1 comprises an inlet end 3, milking region 4, outlet tube 5, outlet end 6 and closure element 7 which is attached to the outlet element 1 by a connector 8. The outlet element 1 is tubular and its tubular shape extends from the inlet end 3, through the milking region 4 and outlet tube 5 to the outlet end 6. As described in more detail below, this provides a fluid path through the outlet element 1 from the inlet end 3 to the outlet end 6 via the milking region 4 and outlet tube 5. The tubular shape of the outlet element 1 defines an axis of the outlet element 1 that extends along the tube from the inlet end 3 to the outlet end 6. The axis is therefore parallel to line C-C in Figure 4.

The inlet end 3 is for connecting to the pouch 37 of an ostomy appliance 2 and receiving stomal output from the pouch 37 to allow draining of the ostomy appliance 2. When viewed down the axis of the outlet element 1, the inlet end 3 is generally ovular, with a lens shape. This provides an optimum shape for front and rear walls 9, 10 of the ostomy appliance 2 and inlet end 3 of the outlet element 1 to be welded together during manufacture as described further below. In this embodiment, the inlet end 3 comprises a major axis of about 5.5 cm extending perpendicular to the axis of the outlet element 1 and parallel to the walls 9, 10. In this embodiment, the inlet end 3 has a minor axis of about 2 cm.

In this embodiment, the inlet end 3 has a length along the axis of the outlet element 1 of about 1 cm. The inlet end 3 has a shape that is substantially constant along this length to provide a tubular inlet for the outlet element 1. The inlet end 3 comprises two flaps 11, one provided on each side of the inlet end 3 along its major axis. The flaps 11 extend outwardly from the outlet element 1 and provide a tapered shape to the lens-shaped inlet end 3 to provide enhanced attachment to the ostomy appliance 2 as described below. In this embodiment, the flaps 11 extend the full length of the inlet end 3.

The milking region 4 extends from the inlet end 3 to the outlet tube 5. In this embodiment, the milking region 4 has a length along the axis of the outlet element 1 of about 4 - 5 cm, or preferably about 4.2 cm. The milking region 4 has a generally ovular cross-section, similar to that of the inlet end 3 but rounded at each end along the major axis and without outwardly extending flaps. Where the milking region 4 connects to the inlet end 3, the major and minor axes of the milking region match that of the inlet end 3. Then along the length of the milking region 4 extending away from the inlet end 3. In this embodiment, the minor axis remains roughly constant at about 2 cm, whereas the major axis tapers down from 5 cm until the major and minor axes are the same where the milking region 4 meets the outlet tube 5.

In this embodiment, the major axis of the milking region 4 reduces linearly over the length of the milking region 4. Consequently, the milking region 4 can be defined by opposing walls 12 that are generally the shape of an inverted trapezium. The slanted edges of the walls 12 being connected to one another to form the milking region 4.

The outlet tube 5 extends from the milking region 4 to the outlet end 6. In this embodiment, the outlet tube 5 has a slightly narrower internal diameter than the milking region 4 where the two parts meet, and as such, an angled step 13 is provided between them. In this embodiment, the outlet tube 5 is then cylindrical with a constant internal diameter of about 1.3 cm to the outlet end 6, which also has an internal diameter of 1.3 cm.

In this embodiment, the milking region 4 has a wall thickness of about 1.5 mm and the inlet has a wall thickness of about 1.7 mm. This helps to ensure the milking region remains relatively flexible compared to the inlet while retaining sufficient strength not to break during use. The similarity in thickness also assists during manufacture and reduces the likelihood of moulding artefacts.

The outlet tube 5 further comprises a pair of internal ridges 14 extending inwards from the inside of the outlet tube 5. These extend all the way around the circumference of the outlet tube 5 and are configured to assist in sealing of objects inserted into the outlet tube 5 as described below. In this embodiment, the internal ridges 14 are positioned approximately one third to a half of the distance from the outlet end 6 to the milking region 4 along the outlet tube 5.

In this embodiment, the outer perimeter or diameter of the outlet tube 5 varies to define different features that enable more efficient connection of devices around the outlet tube 5. At the outlet end 6, the outlet tube 5 comprises a tapered section 15 that is wedge-shaped to assist an external tubular device fitting over and around the outlet tube 5. In this embodiment, the tapered section 15 increases the outer diameter of the outlet tube 5 from the outlet end 6 where it is about 1.5 cm to about 1.7 cm.

The outlet tube 5 also comprises a series of strengthening ribs in the form of four axial ribs 20 and four circumferential ribs 21. The axial ribs 20 extending parallel to the axis of the outlet element 1 and the circumferential ribs 21 extending around the circumference of the outlet tube 5. These ribs 20, 21 are provided on the outer surface of the outlet tube 5 to add strength to it and prevent deformation or collapsing of the tube in use.

In this embodiment, between the ribs 20, 21 and the tapered section 15 there is provided a recessed section 17. In this embodiment, the recessed section 17 has a constant outer diameter of about 1.5 cm. At the interface between the recessed section 17 and the tapered section 15 there is provided a lip 17a. The lip 17a constitutes a step reduction in the outer diameter of the outlet tube 5 of about 14% from the tapered section to the recessed section. This enables the recessed section 17 to better retain a connector of an external drainage device as it can bear against the lip 17a to prevent it from sliding off the outlet tube 5.

At the interface between the recessed section 17 and ribs 20, 21, the outer diameter of the outlet tube 5 is tapered to provide a chamfered edge 18 that extends all the way to the recessed section 17. This makes the outer feel of the outlet element smoother and more comfortable to hold.

In this embodiment, the outlet tube 5 has a length along the axis of the outlet element 1, which is also the axis of the outlet tube 5, of about 2 cm. In this embodiment, the tapered section 15 and recessed section 17 are both about 0.5 cm long, the ribs 20, 21 together cover a length of about 0.5 cm, and the step 13 is also about 0.5 cm. Of course, the sizes are purely exemplary throughout and may be changed or adapted as needed.

The four circumferential ribs 21 are spaced apart along the length of the outlet tube 5 over a length of about 0.5 cm, the rib 21 furthest from the outlet end being at the position along the outlet tube where the connector 8 connects to the outlet tube. The four axial ribs 20 are spaced apart equally around the circumference of the outlet tube 5 and connect the three circumferential ribs 21 closest to the outlet end. These ribs 20, 21 help ensure that when injection moulded, the outlet tube 5 is less susceptible to artifacts caused by large changes in the bulk thickness of the tube while simultaneously increasing the strength of the outlet tube. It also helps optimise the use of raw materials and lowers the weight of the outlet element 1.

The outlet end 6 is configured to be closed by the closure element 7 to inhibit fluid flow out of the outlet element 1. To achieve this, the closure element 7 comprises a tubular plug 22 with a cylindrical shape and outer diameter that matches the internal diameter of the outlet tube 5. The plug 22 has a capped end 23 which may be inserted into the outlet tube 5 to seal the outlet end 6.

To assist insertion of the plug 22 into the outlet tube 5, the capped end 23 is conically shaped, with a truncated point 24 at its tip. In this embodiment, the truncated point 24 has a width of about 1.5 mm and provides a truncated conical shape to the capped end 23. The truncated point 24 therefore covers about 1-2% of the total cross-sectional area of the plug 22/area of the end 23. This helps guide the plug 22 into the outlet end 6 while ensuring there is no sharp point at the end of the plug 22.

To assist sealing between the plug 22 and outlet tube 5, the plug 5 comprises a proximal sealing rib 25 provided around the base of the conical capped end 23 and a further four sealing ribs 26 spaced apart along the length of the plug 22. The sealing ribs 25, 26 have a length measured out from the plug 22, the proximal sealing rib 25 is the shortest and the other ribs 26 become longer the closer they are to the handling element. This is to match a slight narrowing of the outlet tube in direction away from the outlet end.

The sealing ribs 25, 26 span about half the total length of the plug 22 and are provided towards the capped end 23. The sealing ribs 25, 26 extend all the way round the circumference of the plug 22 and are provided at an angle of about 45 degrees pointed away from the capped end 23. This helps to provide efficient sealing of the plug and outlet tube, in conjunction with the internal ribs 14 of the outlet tube 5. The proximal sealing rib 25 is particularly beneficial as it is structurally supported by the capped end 23 to provide better sealing action.

In this embodiment, at the end of the plug 22 distal from the truncated point 24, there is provided a handling element 27. The handling element 27 is tubular and provides a region for the user to grasp or hold in order to move the plug 22 into and out of engagement with the outlet tube 5. The plug 22 and handling element 27 are therefore linearly arranged along the closure element 7.

The handling element 27 has a larger diameter than the plug 22, and as such provides a flat outwardly extending proximal flange 28 around the end of the plug 22 distal from the capped end 23. A surface of the proximal flange 28 facing the plug 22 also provides a stop 29 configured to prevent over insertion of the plug 22 into the outlet tube 5. The stop 29 provides a relatively thick wall region compared to the plug 22 and therefore structurally supports the plug 22 to ensure it works effectively at sealing the outlet tube 5. In this embodiment, the handling element 27 has a wall thickness greater than that of the plug throughout its length to provide additional structural strength to the closure element and plug. For example in this embodiment, the wall thickness of the handling element is about 100% greater than the wall thickness of the plug.

In this embodiment, the proximal flange 28 has an outer diameter of about 2.2 cm, with a rounded outer edge to ensure no sharp edges are felt by the user. From the proximal flange 28, the handling element's shape curves inwards with concave curvature to a cylindrical mid-section 30. In this embodiment, the mid-section 30 comprises an outer diameter of about 1.7 cm and a length of about 1 cm. The shape then curves back outwards with concave curvature to define a distal flange 31 defining an end of the closure element 7 distal from the capped end 23. In this embodiment, the distal flange 31 is larger than the proximal flange 28 and has an outer diameter of about 2 cm. Along the length of the handling element 27, the proximal and distal flanges and mid-section provide a generally flat bottomed U shape that is suitable for comfortably receiving the finger of a user in use as described below.

The distal flange 31 is provided around an opening 32 in the handling element 27. The opening 32 leads to a bore 33 that extends through the handling element 27 and plug 22 where it meets the capped conical end 23. This provides the hollow and tubular shape of the closure element 7. In this embodiment, the closure element 7 therefore comprises a wall that continuously forms the tubular handling element 27 and plug 22 with bore 33 through the middle and closed by the capped end 23.

Due to the change in size of the closure element 7 from the larger handling element 27 to the smaller plug 22, there is a corresponding angled step 34 in the inside of the wall as defined by the bore 33 at a position corresponding to the proximal flange 28.

In this embodiment, the opening 32 is configured to receive the fingertip 44 of a user to assist in movement of the closure element 7 and particularly to help the user disengage the closure element 7 from the outlet tube 5. In this embodiment, the opening 32 is sized to help achieve this, and optimally is 13 mm in diameter (although other sizes and shapes are envisaged). This has been found to provide the appropriate balance between being big enough for most people to be able to insert their finger into the opening while small enough to reduce the need for the user to manually grip or grasp the handling element. For example, due to the finger's natural elasticity, it can be forced into the opening and then will be retained in the opening providing a retention force that can be used to remove the plug from the outlet tube as described further below.

Of course, other sizes and shapes of opening are also envisaged. Where a non-circular opening is used, the maximum width measured between opposite sides of the opening is typically at least 12 mm, or at least 13 mm. For example, where the opening is ovular, the major axis may be at least 12 mm.

In this embodiment, the handling element 27 and the rest of the outlet element 1 are formed from an elastically deformable material, such as the co-polymer ethylene vinyl-acetate (EVA), with a vinyl-acetate concentration of 18% by weight. This can provide a material with a shore A hardness of 90 and a Young's modulus of about 3.6 MPa. The shore A hardness being measured by the ISO 48-4 and/or ASTM D2240 standards. This is optimally suited to providing elasticity that complements that of the finger to ensure the fingertip is "gripped" by the opening 32 and that the opening 32 deforms to match the shape of a user's finger.

In this embodiment, the handling element 27 has a length from the stop 28 to the opening 32 of about 1.8 to 2 cm, or at least the diameter of the opening 32. This helps ensure that the user can easily apply a force to the handling element via their fingertip, that the fingertip may be sufficiently inserted to generate a good gripping force by the opening, and that the opening is sufficiently spaced apart from the outlet tube when connected to it that the user is less at risk of contacting any stomal output that might leak or be present on the outside of the outlet tube.

In addition, due to the larger outer diameter of the distal flange 31 compared to the size of the opening 32, an annular region of elastic material is formed around the opening 32 that is thicker than the wall thickness of the handling element in the neighbouring midsection 30. Consequently, the handling element is relatively rigid around the opening 32 as compared to the midsection 30. This allows a user to force their fingertip through the opening 32 where it is received in the midsection 30, the opening 32 may then provide additional gripping force to help the user remove the closure element from the outlet end as the opening expands less than the midsection to accommodate the fingertip.

In this embodiment, the connector 8 extends parallel to the major axis of the inlet end 3 and parallel to the walls 9, 10 of the ostomy appliance 2. This ensures that the connector 8 can hold the closure element 7 away from the outlet element 1 but in the plane of the walls 9, 10 which makes the overall appliance more compact in a direction perpendicular to the walls 9, 10.

In this embodiment the connector 8 comprises a lateral arm 33 extending from the outlet tube 5 at a connector location 34 corresponding to one of the circumferential ribs 21. The lateral arm 33 extends perpendicular to the axis of the outlet tube 5 and parallel to the major axis of the inlet end 3. In this embodiment, the lateral arm 33 has a length that is about 50% greater than the distance from the connector location 34 to the outlet end 6, this balances the need for a longer connector to enable the plug 22 to be received in the outlet tube 5 while reducing wastage of materials and minimising the overall size of the outlet element 1. Additionally, the plug 22 has a length that is equal to the distance from the connector location 34 to the outlet end 6, this helps ensure the plug 22 is better aligned with the outlet end 6 when it moves into/out of the outlet end 6.

The connector 8 also comprises a curved section formed of an axial arm 35 and second lateral arm 36. The axial arm 35 extends from the lateral arm 33 in a direction parallel to the axis of the outlet tube 5 and towards the inlet end 3. The second lateral arm 36 extends from the axial arm 35 in a direction parallel to the lateral arm 34 and to the closure element 7. In this embodiment, the second lateral arm 36 connects to the closure element 7 at a point in the mid-section 30 adjacent the proximal flange 28. This helps ensure the plug 22 is more intuitively received centrally onto the outlet end 6.

In this embodiment, the lateral arm 33 has a length of about 2-3 cm, the axial arm 35 about 1 cm and the second lateral arm 36 about 0.5-1 cm. Of course, as with all sizes disclosed, these could be varied to suit the specific application. The lateral arm being longer is generally preferred to help provide clearance between the axial arm and milking region 4.

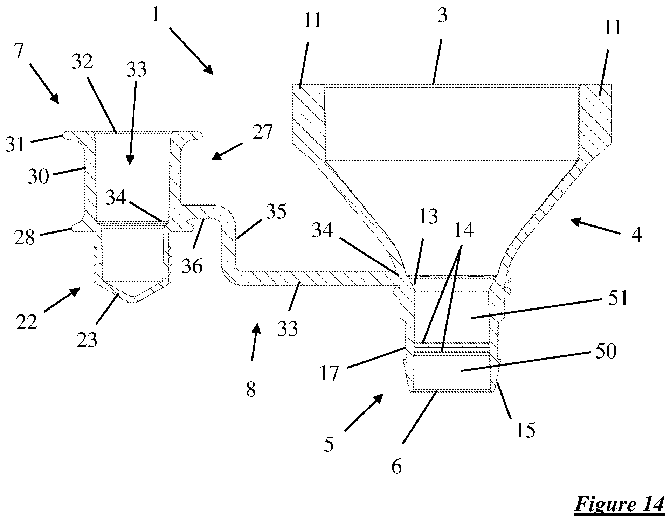

Referring to Figure 14, the outlet tube 5 may comprise a first section 50 and a second section 51. The internal ribs 14 may define a boundary between the first section 50 and second section 51 of the outlet tube 5. In some embodiments, the internal diameter of the second section 51 is less than the internal diameter of the first section 50. This ensures an effective tight seal as the plug 22 is inserted further into the outlet tube 5. In some embodiments, the diameter of the first section may be between 10mm to 20mm, or it may be between 10mm to 19mm, 10mm to 18mm, 10mm to 17mm, 10mm to 16mm, 10mm to 15mm, 10mm to 14mm, 10mm to 13mm, 10mm to 12mm or 10mm to 11mm. In some embodiments, the diameter of the second section is between 10mm to 15mm, or it may be between 10mm to 14mm, 10mm to 13mm, 10mm to 12mm or 10mm to 11mm. In some embodiments, the internal diameter of the first section of the outlet tube is between 10mm to 16mm and the internal diameter of the second section of the outlet tube is between 10mm to 15.5mm. For example, the internal diameter of the first section of the outlet tube may have a minimum diameter of 10.28mm and a maximum diameter of 15.8mm. In another example, the first section of the outlet tube may have a minimum diameter of 12mm and a maximum diameter of 13.2mm, or it may have a minimum diameter of 12.85mm and a maximum diameter of 13.16mm. The internal diameter of the second section of the outlet tube may have a minimum diameter of 10mm and a maximum diameter of 15.5mm, for example a minimum diameter of 10.07mm and a maximum diameter of 15.48mm. In another example, the second section of the outlet tube may have a minimum diameter of 12.5mm and a maximum diameter of 13mm, or it may have a minimum diameter of 12.59mm and a maximum diameter of 12.90mm.

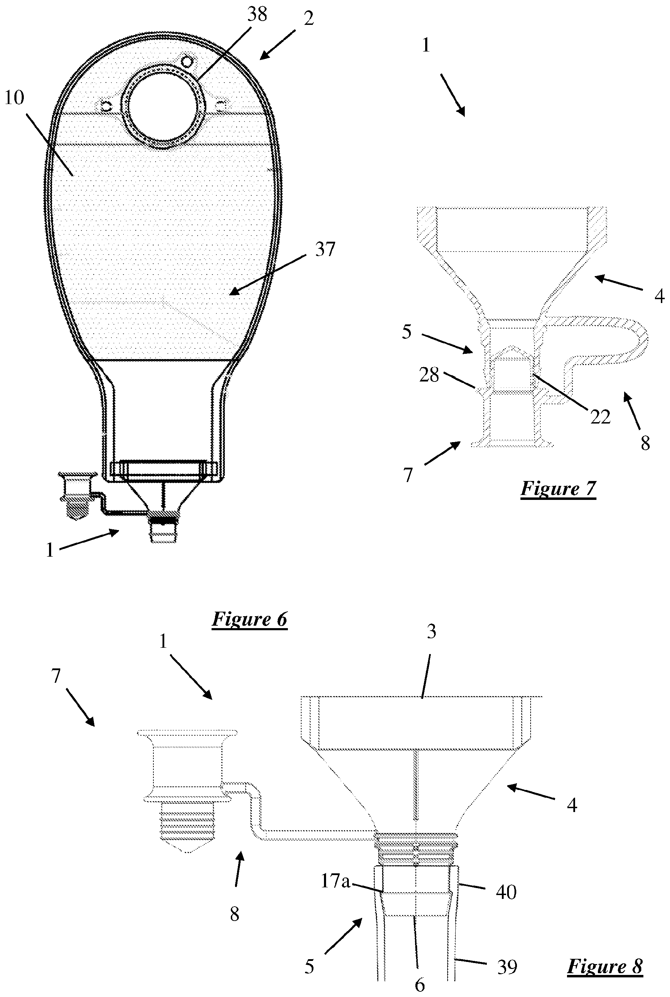

Referring to Figures 6 and 7, the outlet element 1 is provided as part of an ostomy appliance 2. As mentioned above, the ostomy appliance 2 comprises walls 9, 10 sealed around their periphery to form a pouch 37 for receiving stomal output from the user's stoma. In this embodiment, the walls 9, 10 are welded together to form the pouch 37 with the inlet end 3 of the outlet element 1 at the bottom of the pouch 37 to receive stomal output from the pouch 37 as described below.

In this embodiment, the walls 9, 10 comprise a front wall 9 and rear wall 10. The rear wall 10 is intended to face the body of a user in use and comprises coupling element 38 for coupling to an ostomy baseplate (not shown). The ostomy baseplate being configured to attach to the body of a user around their stoma. In other embodiments, the rear wall itself may comprise an adhesive wafer or other device to attach the ostomy appliance directly to the body of a user.

The rear wall 10 also comprises a stomal inlet to receive the stoma of a user and allow stomal output to pass into the pouch 37.

In this embodiment, the front wall 9 comprises a gas vent 39 to allow any stomal gases in the pouch 37 to escape and prevent the pouch 37 from "ballooning" during use.

Injection moulding is preferred as the method of manufacture for the outlet element 1. Due to the generally tubular and hollow design of both the outlet tube 5 and closure element 7, the overall design is suitable for injection moulding in a single step. Of course, other moulding techniques could be used or other manufacturing techniques such as additive manufacturing.

A suitable material for forming the outlet element 1 is the commercially available co-polymer is ELVAX (RTM) 460 Ethylene Vinyl Acetate Copolymer obtained from The Dow Chemical Company and corresponding to the Product Data Sheet with revision date 26 February 2021. The product is melted to form a thermoplastic melt and then injection moulded to form the outlet element 1.

Once the outlet element 1 is formed, the walls 9, 10 of the ostomy appliance may be provided. They may be provided with the coupling element 38 and gas vent 39 already fitted to the rear and front walls respectively as per known manufacturing procedures. Preferably, the walls 9, 10 are formed of a material that may be welded with the materials of the outlet element 1, for example a material comprising EVA and/or VA, or a thermoplastic that is compatible with them. The pouch 37 is then formed by welding around the periphery of the walls with the outlet element 3 positioned between them at the base of the ostomy appliance. This causes the outlet element 3 to be welded to the walls 9, 10 simultaneously with them being welded together around the rest of the pouch 37. In this embodiment, heat welding is used, but of course other welding techniques or attachment techniques could also be used, such as friction welding, laser welding, or attachment with adhesives etc.

In order to use the ostomy appliance 2, it is first fitted to the user's body by coupling it to the baseplate attached to the user. The outlet element 1 may be provided with the plug 22 already closing the end of the outlet tube 4. However, if not, the user can grasp the handling element 27 and insert the plug 22 into the outlet end 6 until the outlet end 6 bears against the stop 29. This will ensure that there are no unintended leaks out of the outlet end 6.

When the pouch 37 needs to be emptied or drained, the user may insert their fingertip into the opening 32. Due to the elasticity of the fingertip and also that of the opening 32, a gripping action is provided between the opening 32 and bore 33 and the fingertip 44. Advantageously, as the opening 32 is spaced apart from the stop 29, the handling element is not directly structurally supported by the outlet tube. This makes stretching of the opening 32 and handling element easier for the user.

The elasticity of the opening 32 may allow it to change shape and/or size to accommodate and grip a user's fingertip. For example, for a relatively small but ovular fingertip, the opening 32 may change from a circular shape to an oval as the ovular fingertip is inserted therein. This will cause a restoring force urging the opening back towards a circular shape that grips the fingertip. In another example, where the fingertip is larger, the opening 32 may just increase in diameter or width, and may also change shape to accommodate the fingertip. The elasticity providing a force to urge the opening back to its original size and thereby "gripping" the fingertip.

Generally, a human finger has an ovular cross-section, and so the opening usually changes shape to accommodate this. Of course, in some embodiments the opening might be ovular to match the cross-section of a finger and so might not change shape.

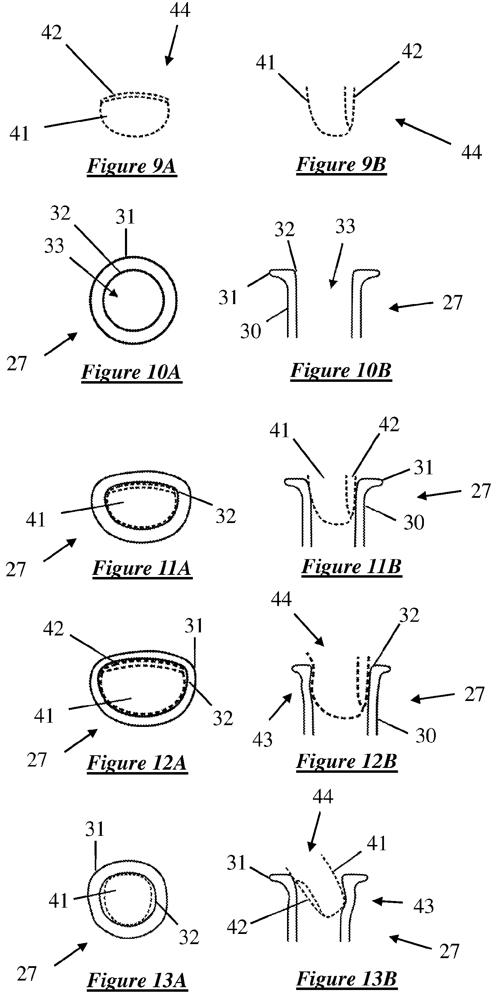

This gripping action allows the user to pull their finger back away from the outlet end 6 and pull the plug 22 out of the outlet end 6 without needing to manually grip the handling element 27 to the same extent as previous outlet elements. To provide additional gripping action, the user can also angle their finger with respect to the axis of the outlet tube 5 to provide additional stretching of the handling element, see Figures 13A and 13B for example as described later. This can also help provide a torque to the handling element to help remove the plug 22 from the outlet tube 5.

If required, the user may also pinch the outside of the mid-section 30 between one finger and the finger inserted into the opening 32. The distal flange 31 can also help the user to apply a force to remove the plug 22 from the outlet tube 5. While doing this, the proximal flange 28 provides a barrier to prevent the user from accidentally touching the outlet tube 5 itself, which may be dirty.

Once the plug 22 is removed from the outlet tube 5, stomal output may pass through the outlet end 6 and leave the ostomy appliance 2. To assist this process, the user can gently squeeze the milking region 4 from the inlet end 3 towards the outlet tube 5 to progressively urge stomal output down and out of the outlet end 6. This can be helpful where the stomal output is a mixture of solids and liquids as is typical for an ileostomy.

Additionally, during this process, the connector 8 holds the closure element 7 out of the way to ensure the user doesn't accidentally touch it while milking and to ensure stomal output passing out of the outlet end 6 doesn't contact the closure element 7.

Once draining is complete, the user can grasp the handling element 27 again, optionally using the opening 32 again, and re-insert the plug 22 into the outlet end 6 to close the outlet tube 5. In this action, the user may place their finger over the opening 32 to press the handling element into the outlet end without their fingertip entering the opening 32. This ensures that the opening 32 does not grip the fingertip which may lead to the plug inadvertently becoming disengaged from the outlet end when the user withdraws their finger.

As described above, the user may wish to drain the ostomy appliance directly into a receptacle such as a toilet. However, they may also wish to connect an external drainage device, such as a drainage tube 39 to handle the stomal output. This can be particularly beneficial for usage at night in combination with a night drainage bag (not shown).

Referring to Figure 8, an example is shown in which a drainage tube 39 is connected to the outlet tube 5. In this embodiment, the drainage tube 39 comprises a cuff region 40 that is configured to grip to the recessed section 17 of the outlet tube 5. This allows the user to insert the outlet tube 5 into the drainage tube 39 so that it tightly fits the recessed section 17 and the lip 17a prevents disengagement between the drainage tube 39 and outlet tube 5. In other embodiments, a locking cuff is provided by an elasticated band, a hose clamp or a device similar to a zip tie, or other kind of pipe coupling device. This can provide more secure engagement if required.

Of course, many other drainage devices could be connected to the outlet tube 5. In another example, an adaptor is connected within the outlet tube. The adaptor facilitates connection of narrower drainage tubing than could otherwise be connected to the outlet tube.

Figure 9A shows an end on view, or axial cross-section, of a user's fingertip 44, with a finger pad 41 forming the fleshy underside of the finger and a finger nail 42 over the top. Figure 9B shows a side view, or side cross-section, of the same fingertip 44. Figures 10A and 10B show corresponding views of the handling element 27 including the midsection 30, distal flange 31, opening 32 and bore 33.

Referring to Figures 11A to 13B, end and side views are shown of three examples of how the opening 32 may be used by different users is shown. In a first example shown in Figures 11A and 11B, the user's fingertip 44 has a similar area to the opening 32 but an ovular shape. As such, when the fingertip 44 is inserted into the opening 32, it changes shape to accommodate the fingertip 44 but does not significantly change in size.

Referring to Figures 12A and 12B, in a second example, the user's fingertip 44 is larger than the opening 32 and also ovular. In this example, the opening 32 changes shape but is also stretched to accommodate the user's fingertip 44. As the midsection 30 is more flexible than the opening 32, due to the thicker material provided by the presence of the distal flange 31 around the opening 32, a bulge 43 is formed in the midsection. This bulge 43 means the user can pull on the opening 32 with greater force to remove the closure element from the outlet tube 5.

Referring to Figures 13A and 13B, in a third example, the user's fingertip 44 is smaller than the opening 32. As such, they insert their finger at an angle and create a bulge 43 by driving the fingertip 44 into the midsection 30 below the opening 32. This enables the user to apply a force to disengage the closure element from the outlet tube 5. In this example, the user hooks the finger pad 41 into and under one side of the opening 32 to be able to pull the handling element 27.

The above examples are enabled in part by the size of the opening but also the depth of the bore to allow the user to insert their fingertip into the opening and the stop which is provided spaced apart from the opening. Having the stop spaced apart ensures that the outlet tube itself does not brace the sides of the midsection 30 which may make it hard or impossible to form an effective bulge 43 as described above.

The one or more embodiments are described above by way of example only.

The scope of the invention, however, is solely defined by the appended claims.

## Claims

1. An outlet element (1) for an ostomy appliance (2), the outlet element (1) comprising an outlet tube (5) for allowing stomal output to exit the ostomy appliance (2) and a closure element (7) configured to releasably close an outlet end (6) of the outlet tube (5) to inhibit release of stomal output from the ostomy appliance (2), wherein the closure element (7) comprises a tubular handling element (27) that provides an opening (32) suitable for receiving the fingertip of a user to assist in disengaging the closure element (7) from the outlet tube (5); **characterised in that** the closure element (7) comprises a plug (22) configured to be received into the outlet tube (5); and wherein the plug (22) and handling element (27) are provided linearly along the closure element (7).

2. The outlet element (1) of claim 1 wherein the opening (32) has a width/diameter of at least 12 mm.

3. The outlet element (1) of claim 2 wherein the opening (32) has a width/diameter of 12 to 20 mm.

4. The outlet element (1) of claim 3 wherein the opening (32) width/diameter is 13 mm.

5. The outlet element (1) of any preceding claim wherein the closure element (7) comprises a stop (29) configured to abut the outlet end (6) of the outlet tube (5) and the opening (32) is spaced apart from the stop (29) by a distance of at least half a diameter of the opening (32).

6. The outlet element (1) of claim 5 wherein the handling element (27) extends from the stop (29) away from the outlet end (6).

7. The outlet element (1) of any preceding claim wherein the plug (22) comprises a conical end (23).

8. The outlet element (1) of any preceding claim wherein the plug (22) comprises one or more sealing ribs (25, 26) configured to seal against an inside of the outlet tube (5).

9. The outlet element (1) of any preceding claim wherein the handling element (27) comprises a bore (33) extending from the opening (32) into the handling element (27); wherein the bore (33) is cylindrical.

10. The outlet element (1) of any preceding claim wherein the handling element (27) comprises an outwardly extending proximal flange (28) at an end adjacent to the outlet tube (5) when the outlet end (6) is closed by the closure element (7); wherein the closure element (7) comprises a stop (29) configured to abut the outlet end (6) of the outlet tube (5) and the proximal flange (28) is positioned around the stop (29).

11. The outlet element (1) of any preceding claim wherein the handling element (27) comprises an outwardly extending distal flange (31) around the opening (32).

12. The outlet element (1) of claim 11, when being dependent on any of claims 1-9, wherein the handling element (27) comprises an outwardly extending proximal flange (28) at an end adjacent to the outlet tube (5) when the outlet is closed by the closure element (7), and the distal flange (31) extends out further than the proximal flange (28).

13. The outlet element (1) of any preceding claim comprising an outwardly extending flange (28, 31), wherein the handling element (27) curves outward to meet the flange (28, 31).

14. The outlet element (1) of any preceding claim comprising a milking region (4) provided between the inlet end (3) and outlet tube (5), wherein the milking region (4) is configured to allow a user to squeeze stomal output towards the outlet tube (5).

15. An ileostomy appliance (2) comprising two walls (9, 10) sealed around their periphery to form a pouch (37) and the outlet element (1) of any preceding claim provided at or towards a bottom of the pouch (37) for draining the contents of the pouch (37).

## Patentansprüche

1. Auslasselement (1) für eine Stomavorrichtung (2), wobei das Auslasselement (1) ein Auslassrohr (5), um zu ermöglichen, dass Stomaausscheidungen aus der Stomavorrichtung (2) austreten kann, und ein Verschlusselement (7), ausgebildet, um ein Auslassende (6) des Auslassrohrs (5) lösbar zu verschließen, um eine Abgabe von Stomaausscheidungen aus der Stomavorrichtung (2) zu hemmen, umfasst, wobei das Verschlusselement (7) ein rohrförmiges Betätigungselement (27) umfasst, das eine Öffnung (32) bereitstellt, geeignet zur Aufnahme der Fingerkuppe eines Benutzenden, um das Lösen des Verschlusselements (7) von dem Auslassrohr (5) zu unterstützen; **dadurch gekennzeichnet, dass** das Verschlusselement (7) einen Stopfen (22) umfasst, ausgebildet, um in dem Auslassrohr (5) aufgenommen zu werden; und wobei der Stopfen (22) und das Betätigungselement (27) linear entlang des Verschlusselements (7) bereitgestellt sind.

2. Auslasselement (1) nach Anspruch 1, wobei die Öffnung (32) eine Breite/einen Durchmesser von mindestens 12 mm aufweist.

3. Auslasselement (1) nach Anspruch 2, wobei die Öffnung (32) eine Breite/einen Durchmesser von 12 bis 20 mm aufweist.

4. Auslasselement (1) nach Anspruch 3, wobei die Breite/der Durchmesser der Öffnung (32) 13 mm beträgt.

5. Auslasselement (1) nach einem vorhergehenden Anspruch, wobei das Verschlusselement (7) einen Anschlag (29), ausgebildet, um an dem Auslassende (6) des Auslassrohrs (5) anzuliegen, umfasst und die Öffnung (32) von dem Anschlag (29) um einen Abstand von mindestens einem halben Durchmesser der Öffnung (32) beabstandet ist.

6. Auslasselement (1) nach Anspruch 5, wobei sich das Betätigungselement (27) von dem Anschlag (29) von dem Auslassende (6) weg erstreckt.

7. Auslasselement (1) nach einem vorhergehenden Anspruch, wobei der Stopfen (22) ein konisches Ende (23) umfasst.

8. Auslasselement (1) nach einem vorhergehenden Anspruch, wobei der Stopfen (22) eine oder mehrere Dichtungsrippen (25, 26), ausgebildet, um gegen eine Innenseite des Auslassrohrs (5) abzudichten, umfasst.

9. Auslasselement (1) nach einem vorhergehenden Anspruch, wobei das Betätigungselement (27) eine sich von der Öffnung (32) in das Betätigungselement (27) erstreckende Bohrung (33) umfasst; wobei die Bohrung (33) zylindrisch ist.

10. Auslasselement (1) nach einem vorhergehenden Anspruch, wobei das Betätigungselement (27) einen sich nach außen erstreckenden proximalen Flansch (28) an einem Ende benachbart zu dem Auslassrohr (5) umfasst, wenn das Auslassende (6) durch das Verschlusselement (7) verschlossen ist; wobei das Verschlusselement (7) einen Anschlag (29), ausgebildet, um an dem Auslassende (6) des Auslassrohrs (5) anzuliegen, umfasst und der proximale Flansch (28) um den Anschlag (29) herum positioniert ist.

11. Auslasselement (1) nach einem vorhergehenden Anspruch, wobei das Betätigungselement (27) einen sich nach außen erstreckenden distalen Flansch (31) um die Öffnung (32) herum umfasst.

12. Auslasselement (1) nach Anspruch 11, wenn von einem der Ansprüche 1 bis 9 abhängig, wobei das Betätigungselement (27) einen sich nach außen erstreckenden proximalen Flansch (28) an einem Ende benachbart zu dem Auslassrohr (5) umfasst, wenn der Auslass durch das Verschlusselement (7) verschlossen ist, und der distale Flansch (31) sich weiter nach außen erstreckt als der proximale Flansch (28).

13. Auslasselement (1) nach einem vorhergehenden Anspruch, umfassend einen sich nach außen erstreckenden Flansch (28, 31), wobei sich das Betätigungselement (27) nach außen krümmt, um an den Flansch (28, 31) anzuschließen.

14. Auslasselement (1) nach einem vorhergehenden Anspruch, umfassend einen Ausstreichbereich (4), bereitgestellt zwischen dem Einlassende (3) und dem Auslassrohr (5), wobei der Ausstreichbereich (4) ausgebildet ist, um einem Benutzer zu ermöglichen, Stomaausscheidungen in Richtung des Auslassrohrs (5) zu drücken.

15. Ileostomavorrichtung (2), umfassend zwei Wände (9, 10), abgedichtet um ihren Umfang herum, um einen Beutel (37) zu bilden, und das Auslasselement (1) nach einem vorhergehenden Anspruch, bereitgestellt an oder in Richtung eines Bodens des Beutels (37), um den Inhalt des Beutels (37) abzulassen.

## Revendications

1. Un élément (1) de sortie d'un appareil (2) de stomie, l'élément (1) de sortie comprenant un tube (5) de sortie pour permettre à de la sortie stomale de sortir de l'appareil (2) de stomie et un élément (7) de fermeture configuré pour fermer, avec possibilité de libération, une extrémité (6) de sortie du tube (5) de sortie pour empêcher une libération de sortie stomale de l'appareil (2) de stomie, dans lequel l'élément (7) de sortie comprend un élément (27) tubulaire de manipulation, qui donne une ouverture (32) propre à recevoir la pointe du doigt d'un utilisateur pour faciliter le désengagement de l'élément (7) de fermeture du tube (5) de sortie ; **caractérisé en ce que** l'élément (7) de fermeture comprend un bouchon (22) configuré pour être reçu dans le tube (5) de sortie ; et dans lequel le bouchon (22) et l'élément (27) de manipulation sont prévus linéairement le long de l'élément (7) de fermeture.

2. L'élément (1) de sortie de la revendication 1, dans lequel l'ouverture (32) a une largeur/diamètre d'au moins 12 mm.

3. L'élément (1) de sortie de la revendication 2, dans lequel l'ouverture (32) à une largeur/diamètre de 12 à 20 mm.

4. L'élément (1) de sortie de la revendication 3, dans lequel l'ouverture (32) a une largeur/diamètre de 13 mm.

5. L'élément (1) de sortie de l'une quelconque des revendications précédentes, dans lequel l'élément (7) de
. fermeture comprend une butée (29) configurée pour buter sur l'extrémité (6) de sortie du tube (5) de sortie et l'ouverture (32) est à distance de la butée (29) d'une distance d'au moins la moitié d'un diamètre de l'ouverture (32).

6. L'élément (1) de sortie de la revendication 5, dans lequel l'élément (27) de manipulation part de la butée (29) en s'éloignant de l'extrémité (6) de sortie.

7. L'élément (1) de sortie de l'une quelconque des revendications précédentes, dans lequel le bouchon (22) comprend une extrémité (23) conique.

8. L'élément (1) de sortie de l'une quelconque des revendications précédentes, dans lequel le bouchon (22) comprend une ou plusieurs nervures (25, 26) d'étanchéité configurées pour s'appliquer avec étanchéité sur une face intérieure du tube (5) de sortie.

9. L'élément (1) de sortie de l'une quelconque des revendications précédentes, dans lequel l'élément (27) de manipulation comprend un alésage (33) s'étendant à partir de l'ouverture (32) dans l'élément (27) de manipulation ; dans lequel l'alésage (33) est cylindrique.

10. L'élément (1) de sortie de l'une quelconque des revendications précédentes, dans lequel l'élément (27) de manipulation comprend un rebord (28) proximal s'étendant vers l'extérieur à une extrémité voisine du tube (5) de sortie, lorsque l'extrémité (6) de sortie est fermée par l'élément (7) de fermeture ; dans lequel l'élément (7) de fermeture comprend une butée (29) configurée pour venir en butée sur l'extrémité (6) de sortie du tube (5) de sortie
. et le rebord (28) proximal est en position autour de la butée (29).

11. L'élément (1) de sortie de l'une quelconque des revendications précédentes, dans lequel l'élément (27) de manipulation comprend un rebord (31) distal s'étendant vers l'extérieur autour de l'ouverture (32).

12. L'élément (1) de sortie de la revendication 11, lorsqu'elle dépend de l'une quelconque des revendications 1 à 9, dans lequel l'élément (27) de manipulation comprend un rebord (28) proximal s'étendant vers l'extérieur à une extrémité voisine du tube (5) de sortie, lorsque la sortie est fermée par l'élément (7) de fermeture, et le rebord (31) distal s'étend vers l'extérieur plus loin que le rebord (28) proximal.

13. L'élément (1) de sortie de l'une quelconque des revendications précédentes, comprenant un rebord (28, 31) s'étendant vers l'extérieur, dans lequel l'élément (27) de manipulation s'incurve vers l'extérieur pour rencontrer le rebord (28, 31).

14. L'élément (1) de sortie de l'une quelconque des revendications précédentes, comprenant une région (4) de traite prévue entre l'extrémité (3) d'entrée et le tube (5) de sortie, dans lequel la région (4) de traite est configurée pour permettre à un utilisateur de presser de la sortie stomale vers le tube (5) de sortie.

15. Un appareil (2) d'iléostomie comprenant deux parois (9, 10) scellées autour de leur périphérie pour fermer une poche (37) et l'élément (1) de sortie suivant l'une quelconque des
. revendications précédentes prévu à ou ouvert à un fond de la poche (37) pour drainer les contenus de la poche (37).
.
